# EUROPEAN PATENT SPECIFICATION

(11) **EP 0 438 929 B1**
(45) Date of publication and mention of the grant of the patent: **29.09.1993**
(21) Application number: 90400175.7
(22) Date of filing: 22.01.1990
(51) Int. Cl.: A61K 37/36, A61K 9/51, A23K 1/165

(54) **A composition based on somatotropin for controlling the sea water adaptation of farmed salmonids**
Mittel auf Somatotropinbasis zur Steuerung der Meereswasseradaptation von Zuchtlachsen
Composition à base de somatotropine pour contrôler l'adaptation des saumons d'élevage à l'eau de mer

(43) Date of publication of application: 31.07.1991
(73) Proprietor: EUROGENTEC S.A., 4102 Seraing (BE)
(72) Inventor: Smal, Jean, B-4900 Angleur (BE); Renard, André, B-4900 Liege (BE)
(74) Representative: Gutmann, Ernest

(56) References cited:
- EP-A- 0 157 423
- EP-A- 0 213 357
- EP-A- 0 330 180
- EP-A- 0 353 045
- CHEMICAL ABSTRACTS, vol. 102, no. 23, 10 June 1985, Columbus, OH (US); S.MIWA et al., p. 383, no. 201437w.

## Description

The present invention relates to a somatotropin treatment for controlling the sea water adaptation of farmed salmonids.

Salmonids as contemplated herein are a fish family characterized by a life cycle involving both fresh water and sea water phases. Spawning and early life occur in fresh water. Premature transfer to sea water at that stage is lethal. Most salmonids species reach in the course of their life cycle a stage known as the smolt stage which confer on the fish the capability of surviving in the sea, before it migrates into the ocean where most of its growth occurs.

This parr-smolt transformation (smoltification) is a critical step in the life cycle of the fish and thus in its farming (the aquaculture of trout and salmon is one of the major intensive aquaculture in the world). The production of so-called S2 or S1 smolts (within less than 3 or years from the date they hatched) or if possible SO smolts (within less than 1 year from hatching) is regarded as highly desirable in salmonid farming. The results usually reported for Atlantic salmon farming are of less than 10 % SO smolts, 40 to 70 S1 smolts and the remain being S2 smolts.

The smoltification process involves complex physiological, anatomical and behavioural changes. The fish loses its young parr marking and becomes silvery in colour. Scales become loose and make the smolt prone to injury. Feeding decreases and there is a fall in condition factor as the fish becomes longer and thinner. The swim bladder becomes larger and the fish becomes restless, shoaling in midwater, adapting a schooling behaviour. One of the most characteristic features of the smolt is its adaptation to the high salinity (>28 %o) of sea water. The chloride cell number in the gills and the associated NA⁺K⁺AT-Pase activity increase, the urine production by the kidney declines and the fish is then capable of transfer to sea water.

These changes are triggered by changes in water temperature and day length, and involves numerous hormonal changes. Although thyroid hormones have been implicated in the control of smoltification (Fol- mar and Dickhoff, Aquaculture, 23, 309:324, 1981 ; Grau et al, in "Current Trends in comparative endocrinology" B. Lofts and Holmes eds., Univ. Press Hong Kong, 1985; Barron, J. Endocrinol. 108, 313:319, 1986), evidence for a precise role of thyroxine and triiodothyronine in the acquisition of seawater adaptability is not yet forthcoming (Boeuf, PhD thesis, Uni- versite de Bretagne Occidentale, France, 1987). Increasing evidence suggests that cortisol, another important hormone in teleost osmoregulation, causes physiological changes associated with seawater adaptation (Speckerand Schreck, Gen. Comp. Endocrinol., 46, 53:58, 1982 ; Barton et al, Gen. Comp. Endocrinol, 59, 468:471, 1985 ; Virtanen and Soivia, Aquaculture, 45, 97:109, 1985; Young et al, Gen. Comp. Endocrinol., 74, 346:374,1986; Richman and Zaugg, Gen. Comp. Endocrinol., 65, 189:198, 1987 ; Bjornsson et al, Gen. Comp. Endocrinol., 68, 421:430, 1987). However, in Atlantic salmon, plasma cortisol does not appear to be directly related to the incrase in grill Na⁺K⁺ATPase activity observed during smoltification (Langhorne and Simpson, Gen. Comp. Endocrinol., 61, 203-213, 1986).

Although the importance of pituitary hormones in salmonid osmoregulation is well documented, few studies have focused on this aspect during smoltification. Several studies indicate that prolactin (PRL) is not necessary for fishwater survival of hypophysec- tomized salmonids (Komourdjian and Idler, Gen. Comp. Endocrinol., 32, 536:542, 1977; Bj6rnsson and Hanson, Gen. Comp. Endocrinol., 49,240:247,1983; Nishioka et al, Aquaculture, 65, 343:352,1987). However, measurement of plasma PRL levels in salmonids transferred from fresh water to seawater or the converse support a possible role of PRL as a freshwater adapting hormone (Hirano et al, Gen. Comp. Endocrinol., 59, 266:276, 1985 ; Prunet et Boeuf, Aquaculture, 45, 167:176, 1985 ; Prunet et al, Gen. Comp. endocrinal., 74, 355:364, 1989). Somatropin (ST) may also be implicated in the control of seawater adaptation. Different authors have reported a rise in plasma GH levels during the smoltification of Coho salmon (Sweeting et al, Aquaculture, 45, 185:197, 1985; Comp. Biochem. Physiol., 88, 147:151, 1987 ; Young et al, Gen. Comp. Endocrinol., 74, 346:374, 1989) and Atlantic salmon (Prunet et al, 1989, ibid.). In addition, histological studies have shown that the somatotroph cells increase in size, number and apparent activity upon exposure of Coho smolts to seawater (Clarke et al, Canad. J. Zool., 55, 1620:1630, 1977 ; Nishioka et al, Aquaculture 28, 21:38, 1982 ; Nagahama et al, Can. J. Zool., 55, 183:198, 1977). Neverthless it was found that a rise in GH levels early in seawater adaptation does not appear to have a direct effect on early Na⁺K⁺ATPase activity (Sweeting et al, 1987, ibid.; Smith et al, Mem. Soc. Endocr., 5, 83:101, 1956 ; Komourdjian et al, Canad. J. Zool., 54, 531:535, 1976 ; Clarke et al, 1977, ibid.; Miwa et al, Gen. Comp. Endocr., 58, 436:442, 1985 ; Bolton et al, J. Endocr., 112,63:68, 1987).

EP 0157423 A2 discloses a purified polypeptide hormone obtained from the pituitary gland of salmonids, which hormone is said to stimulate growth in teleosts.

EP 0213357 A3 relates to a method for culturing fishes by using a special food consisting of a fish growth hormone carried on a water-insoluble carrier.

The article titled "Effects of L-Thyroxine and Ovine Growth Hormone on Smoltification of Amago Salmon (Oncorhynchus rhodurus)" of S.Miwa and Y. Inui published in "General and Comparative Endocrinology" 58, 436-442 (1985), discloses that a combination of L-Thyroxine and ovine growth hormone favor smoltification of amago salmon when injected weekly in the fishes over a period of 72 days.

EP 0353045 A2 discloses sustained release formulations and methods for increasing the growth rate or feed efficiency of poultry roasters by parenteral administration of an effective somatotropin.

The invention stems from the discovery that somatotropin was in itself capable, when maintained at an enhanced level in salmonid species over a relatively limited period of time, of providing the fishes with a considerably improved capability of adapting to seawater not only during the above-mentioned relatively limited periods of time but thereafter too, independently of photoperiodism, water temperature and parr size.

This invention relates to a composition for use mainly by intra-peritoneal injection or oral treatment and authorizing, when administered to salmonids, for the slow release of somatotropin in vivo.

Somatotropin for use in this invention refers to a family of proteins having molecular weights of around 22 Kd produced by the pituitary gland of vertebrate, including fish, involved in the regulation of anabolic processes, and related to the Growth Hormone family defined in mammalian species (Nicoll, Endocr. Rev., 7, 169:203, 1986) ; such hormone has been characterized in Chum salmon (Kawauchi et al, Arch. Biochem. Biophys., 244, 542:552, 1986), Coho salmon (Nicoll et al, Gen. Comp. Endocrinol., 68, 387:399, 1987) and trout (Rentier et al, DNA, 8, 119:127, 1989) ; these proteins can specifically bind to the receptors of the natural somatotropin isolated from the pituitary gland of trout or salmon and present in the membrane of trout or salmon liver cells ; the affinity of the somatotropin being at least 2 % of the affinity of the natural hormone as measured by a radioreceptor assay (such an assay has been described in Fryer, Gen. Comp. Endocrinol., 39, 123:130, 1979).

Preferably the Somatotropin for use in the composition of this invention are salmonid somatotropin.

Purified salmonid Somatotropin (pST) refers to a preparation of hormone which has been enriched in salmonid Somatropin protein using at least one purification step (for injection and oral dosage) or to a recombinant microorganism which expresses in its cytoplasm a high amount of salmonid Somatotropin (for oral dosage only). Preferably, the somatropin used is of identical or homologous to that of the fish administered with it : e.g. natural trout somatotropin or recombinant somatotropin having, as far as the somatotropin part thereof is concerned, substantially the same aminoacid sequence as the natural trout somatotropin, when said somatotropin is intended for administration to trout. But this is not an essential condition. For instance trout may be administered with purified somatotropin originating from salmon or tila- pia, or even with somatotropin of mammalian origin, e.g. bovine somatotropin. Recombinant somatotropin can be substituted for the natural ones. The same observations extend to fish species other than trout, e.g. salmon.

The process of the invention for the control of seawater adaptation of farmed salmonids, when the latter are transferred from fresh water to seawater and are caused to undergo smoltification, comprises administering to said salmonids a composition of somatotropin, preferably a salmonid somatotropin, in association with a physiologically acceptable carrier material selected so as to exert a reversible encapsulation, adsorption or entrappment of said somatotropin and to allow for the sustained and delayed release in vivo of the somatotropin, wherein the amounts of somatotropin and, where appropriate, of the carrier are adjusted so as to enable a survival rate of at least 50 % of the salmonids initially maintained in fresh water, one month after either the end of the administration of the composition or the smoltification, whichever is the latest.

A preferred composition is in a liquid form or sufficiently fluid or dispersed form to allow its injection in fish.

The invention makes more particularly use of a composition administrable to fish wherein the initial concentration of somatotropin is adjusted both relative to the sizes of the fishes for which they are intended and relative to the amounts which can be administered to them by the appropriate route, at a level sufficient as to provide for sustained release, over about seven days of amounts or more, of somatotropin suitable to cause the serum concentration of somatotropin in said fishes to be upheld at a substantially permanent level, e.g. above 3 ng, for instance 5 ng per gram of fish, sufficient to enable a 50 % survival rate of the fishes initially maintained in fresn water one month after either the end of the administration of the composition or the smoltification whichever is the latest.

Generally speaking the smoltification (transfer from fresh water to sea water) is performed approximately simultaneously or just after the end of the treatment.

Suitable compositions to be administered orally (e.g. together with food) or parenterally contain an amount of somatotropin adjusted relative to the fish species to which it is to be administered in a range from 50 ng to 2 µg per gram of fish.

Additional preferred conditions, particularly for use in combination with the features recited above, which ought to be fulfilled are indicated hereafter in connection with examples.

One way to produce a pST is to start with pituitary glands isolated from salmon ortrouts, to mechanically break the cells and to extract the hormone in suitable buffer, to purify the hormone by chromatography as described for example in Kawauchi et al., 1987, ibid. Other conventional enrichment and purification techniques may be used. The final preparations contain preferably at least 10 % by weight of ST as defined by polyacrylamide gel electrophoresis in presence of SDS (SDS PAGE), has no residual toxicity forfish and is characterized by a high potency of osmoregulation in terms of Na⁺K⁺ATPase activity stimulation and/or fish survival after transfer of treated animal in seawater.

Another way to produce the pST is to start with a bacterial or yeast recombinant clone containing a plasmid with the ST gene, obtained by recombinant DNA technology, behind an adequate promotor allowing the high level expression of the protein into the cytoplasm, or, if a signal peptide is used, into the periplasm or the fermentation medium as described for example, in European Patent Filing 894000047.0 (1989). If the hormone is produced as an insoluble material, it can be purified, after breaking of the cells, e.g. by a centrifugation step followed by denaturation in a strong chaotropic agent, dialysis and/or chromatographic techniques (e.g. as precedently described as described in Langley et al, Eur. J. Biochem., 163, 313:321, 1987). The final product which contains preferably at least 20 % by weight of ST as characterized by SDS PAGE and is characterized by a low level of residual toxicity for fish and a high potency of osmoregulation in terms of NaKATPase activity stimulation and/or fish survival after transfer of treated animal in seawater. The denaturation step may be omitted if the initial preparation is directly bioactive. This is generally what is observed when the protein is excreted into the periplasm of the microorganism or into the fermentation medium. To help and to facilitate the cloning, expression, renaturation and purification of the protein, such recombinant hormone may contain additional amino acids at the carboxy- and amino-terminus of the protein or may be mutated as far as the modifications are not preventing the binding of the hormone on its receptors from the liver membranes of Salmo salar and/or Oncorhynchus mykiss (similar test is described in Fryer, Gen. Comp. Endocrinol., 39, 123:130, 1979).

The same recombinant microorganisms as described above, but preferably with a cytoplasmic expression of the somatotropin representing preferably at least 0.1 % (w/w) of the total protein, may be used as a "purified salmonid Somatotropin preparation" when the product is to be orally delivered and when the microorganism itself which has been used as the host cell for the production of the recombinant somatotropin, has no pathogenic activity with respect to the salmonid to be treated and, accordingly may be added to the fish food. In that specific case, polymeric encapsulation, such as described below, may be omitted when the capsular membrane of the recombinant microorganism offers a protective coat for the Somatotropin against the denaturing and proteolytic activities present into the gastro-intestinal tract of fish.

The encapsulation of the pST is used to slowly release the hormone and to protect the hormone in the fish intra-peritoneal cavity against proteolytic attack and also, in the case of an oral dosage, against the denaturing and proteolytic activities of the gastro-intestinal tract of the treated fish.

Several systems have already been tested relative to their ability to sustain the release of peptides and proteins. These systems are adaptable to the Production of the ST slow-release compositions of the invention. They include nondegradable and degradable, physiologically acceptable polymeric systems, liposomes and osmotic pumps.

The first attempt to use polymers to sustain the release of macromolecules dated back to 1972, when Davis reported the use of polyacrylamide as a vehicle for insulin (Experentia, 28, 348, 1978). Shortly thereafter Langerand his collaborators began a systematic investigation of ethylene-vinyl acetate copolymer (EVAc) as a alternative protein carrier to the less biocompatible polyacrylamide (Langer and Folkman, Nature, 263, 797:800, 1976 ; Gimbrone et al, J. Natl. Cancer Inst., 52, 413:427, 1974). Their delivery system typically consists of a dispersion of protein particles embeded in an EVAc matrix in the form of either film (Rhine et al, J. Pharm. Sci., 69, 265:270, 1980) or microspheres (Sefton et al, J. Pharm. Sci., 73, 1859:1861, 1984). The protein is released by diffusion through a network of pores and channels created by the dissolving protein particles.

Compared with non-biodegradable systems, biodegradable systems are attractive in that they will not persist in the animal. Systems made of cross-linked serum albumin, poly(lactic acid) (PLA), and poly(d,1- lactide-co-glycolide) (PLGA) have been carefully investigated for peptide and protein delivery (Kwong et al, J. Controlled Release, 4, 47:62, 1986 ; Sanders et al, J. Pharm. Sci., 73,1294:1297,1984; 75, 356:360,1986 ; Goosen et al, Diabetes, 32, 478-481, 1983). However, others like poly(orthoester), poly(anhydride), poly(p-hydroxybutyric acid), and polyphosphazene may also be considered (Heller, Ann. N.Y. Acad. Sci., 446, 51:66, 1985 ; Leong et al, J. Biomed. Mater. Res., 19, 941:955, 1985 ; Grolleman et al, J. Controlled Release, 3, 143-154, 1986, Juni et al, J. Controlled Release, 4, 25:32, 1986). The potential of degradable polymers as controlled release systems for macromolecules has been reviewed (Holand et al, J. Controlled Release, 4, 155:180, 1986). PLGA and homopolymers of lactic and glycolic acids are attractive biodegradable polymers for controled drug delivery because they and their degradation products are biocompatible.

Liposomes have been considered for controlled delivery of proteins for almost two decades (Gurari-Rotman and Lelkes, Biochem. Biophys. Res. Com- mun., 107, 136:143, 1982 ; Weiner t al, J. Pharm. Sci, 74, 922:925, 1985 ; Eppstein et al, Proc. Natl. Acad. Sci, USA, 82, 3688:3692, 1985). Sophisticated multilamellar encapsulation techniques based on liposomes have also been described (in "Liposomes : from physical structure to therapeutic applications", Elsevier, 1981).

The principal strategy for the oral delivery of peptides and proteins has been the protection of a given peptide or protein from the proteolytic enzymes in the lumen of the gastrointestinal tract. Existing oral delivery systems consist in water insoluble gel matrix directly entrapping the protein (in "Controlled Release Technologies ; Methods, Theories and Applications", Kydonieus eds., CRC Press, 1980). Amore recent approach is the encapsulation of insulin with an impervious azopolymer film that remains intact in the gastrointestinal tract until reaching the last intestine (Saf- fran et al, Science, 233,1081:1084,1986). Salmonids are well suited to this approach, since the capacity of distal part of their intestine to absorb intact soluble proteins or large aggregats of proteins is much higher than what is observed in higheranimal species (Geor- gopoulou et Vernier, Cell Tissue Res., 251, 145:152, 1968).

The encapsulation method will be such that :
- it is not denaturing the pST as measured by the binding capacity of the released material to the receptors present on the liver membranes of Salmo salar and or Oncorhynchus mykiss,
- it slowly releases the pST into the fish at a temperature between 1 and 16°C for a period of at least 1 week, the kinetics of the release being such that the amount of pST in the fish during that period is greater than 5 ng per ml of blood serum, as measured by the radioimmunoassay (RIA) described by Bolton et al, Gen. Comp. Endocrinol. (1986), 62, 230-238 ;
- it gives non aggregative microparticles which may be injected with the same equipment as the one classically used for the vaccination of small trout or salmon (e.g. average diameter of the particles between 100 nm and 500 µm), preferably from 100 nm to 1 µ.
- the particles will contain from 0.01 to 10 % (w/w) of somatotropin,
- the polymeric material and the residual reagents used for the encapsulation are not toxic for the fish.

Five different basic methods for microencapsula- tion have been developed ; coacervation, phas separation, interfacial polymerization, electrostatic and mechanical. Although there are many more methods and devices reported and patented for microencapsu- lation, they are usually part of or a combination of those basic methods (Sparks, Encyclopedia of chemical technology (Kirk-Othmer eds), 15, 470:493, 1981).

The invention relates to the treatment (or to compositions specially adapted for the treatment) of salmonids maintained in fresh water by administration of encapsulated salmonid Somatotropin by i.p injection concomitantly or not with vaccine injection or by addition to the fish feed. The consequence of this treatment is the adaptation within 2 weeks to seawater transfer as characterized by
- a survival rate one month after the treatment greater than 50 %,
- a sustained growth rate after the transfer (less than 10 % of stunted animals),
- insensitivity to the diseases and injuries naturally associated with the smoltification process.

Similartransferwithout treatment and outside the natural smoltification period will lead to high mortality (greater than 90 %) and stunted animals.

Typical treatment regimes will be
- one intra-peritoneal injection of 50 ng to 2 µg per g of fish of encapsultated salmonid somatotropin, e.g. 1 % by weight in a poly(decyl- lactide-co-glycolide (PGLA) polymer releasing system, concomitantly or not with vaccines usually injected before transfer to seawater (e.g. vibrio vaccine ; automatic injection machines may be used) ;
- for oral administration, utilization of a Somatotropin supplemented feed for at least one week, the concentration of the Somatotropin in the food being comprised between 10 to 1000 ppm.

For example, sawater adaptation of Salmo salar (20-80 g) can be obtained during periods of the year (July to April) where natural smoltification does not occur, by an i.p. injection of 10 µg of purified recombinant trout somatotropin produced in bacteria and encapsulated in an PLGA polymer (2 mg in 200 µl of saline buffer), with a survival rate greater than 90 % and with less than 5 % of stunted animals.

## Claims

1. A process for the control of seawater adaptation of farmed salmonids, when the latter are transferred from fresh water to seawater and are caused to undergo smoltification, which comprises administering to said salmonids a composition of somatotropin, preferably a salmonid somatotropin, in association with a physiologically acceptable carrier material selected so as to exert a reversible encapsulation, adsorption or entrappment of said somatotropin and to allow for the sustained and delayed release in vivo of the somatotropin, wherein the amounts of somatotropin and, where appropriate, of the carrier are adjusted so as to enable a survival rate of at least 50 % of the salmonids initially maintained in fresh water, one month after either the end of the administration of the composition or the smoltification, whichever is the latest.

2. The process of claim 1, wherein the amount of somatotropin is adjusted both relative to the sizes of the salmonids and to the volume of composition administered to them by the selected route, so as to provide for sustained release, over a period of about seven days, and to cause the serum concentration of somatotropin in said salmonids to be upheld at a substantially permanent level, e.g. higher than 3 ng, for instance 5 ng per gram of fish, during that period.

3. The process of claim 2, wherein the carrier material is selected and wherein the initial concentration of somatotropin is adjusted so as to provide for the above said sustained release in vivo at a temperature between 1° and 16°, the kinetics of the release being such that the amount of pST in the fish during that period is greater than 5 ng per ml of blood serum, as measured by the radioimmunoassay (RIA) described by Bolton et al, Gen. Comp. Endocrinol. (1986), 62, 230-238.

4. The process of any of claims 1 to 3 wherein said composition contains from 50 ng to 2 µg of somatotropin per gram of fish.

5. The process of any of claims 1 to 4 wherein said somatotropin is entrapped in particulate nondegradable and degradable, physiologically acceptable polymeric systems, liposomes or osmotic pumps.

6. The process of claim 5 wherein said carrier material is in the form of particles, having particularly average diameter ranging from 100 nm to 500 µm, preferably from 500 nm to 1 µm.

7. The process of claims 6 or 7 wherein the particulate carrier is formed of a poly(d,l-lactide-co- glycol ide).

8. The process of any of claims 5 to 7 wherein the particles contain from 0.01 to 10 % (w/w) of somatotropin.

9. The process of any of claims 1 to 8 wherein said somatotropin is a recombinant somatotropin having substantially the same aminoacid sequence and the same activity as the natural somatotropin, particularly the salmonid somatotropin corresponding to the salmonid species to which said composition is administered.

10. The process of claim 9 wherein the amount of somatotropin is adjusted in an amount from 50 ng to 2 µg per gram of fish to which the somatotropin composition is administered.

11. The process of any of claims 1 to 9 wherein said composition is in a liquid form or sufficiently fluid or dispersed form to allow its injection in fish and wherein said administration is through injection.

12. The process of any of claims 1 to 9 wherein said composition is administered by the oral route.

13. The process of claim 12 wherein said composition is supplied to said salmonids in admixture with food.

14. The process of claim 13 in combination with claim 9, wherein said composition is in the form of the non-pathogenic host-cell microorganism in which said recombinant somatotropin has been formed and wherein said recombinant somatotropin represents preferably 0.1 % (w/w) of the total protein.

## Patentansprüche

1. Verfahren zum Kontrollieren der Meerwasseradaption von Zuchtsalmoniden beim Transferieren derselben von Süßwasser in Meerwasser, wobei diese zur Smoltifikation veranlaßt werden, mit den Schritten der Verabreichung einer Somatotropinzusammensetzung an die Salmoniden, vorzugsweise eines Salmonidensomatotropins, zusammen mit einem physiologisch verträglichen Trägermaterial, welches so ausgewählt ist, daß es eine reversible Einkapselung, Adsorption oder Einschließung des Somatotropins ausübt und die langandauernde und verzögerte in-vivo-Freisetzung des Somatotropins gestattet, wobei die Menge des Somatotropins und gegebenenfalls des Trägers so eingestellt ist, daß eine Überlebensrate von mindestens 50 % der anfänglich in Süßwasser gehaltenen Salmoniden einen Monat nach entweder dem Ende der Verabreichung der Zusammensetzung oder der Smoltifikation, bezogen auf das jeweils spätere Ereignis, erreicht wird.

2. Verfahren nach Anspruch 1, bei dem die Menge des Somatotropins sowohl relativ zu der Größe der Salmoniden als auch zu dem Volumen der an diese auf dem ausgewählten Wege verabreichten Zusammensetzung eingestellt ist, so daß eine ständige Freisetzung über einen Zeitraum von etwa 7 Tagen geschaffen wird und eine Serumkonzentration von Somatotropin in den Salmoniden auf einem im wesentlichen fortwährenden Niveau, z.B. mehr als 3 ng, insbesondere z.B. 5 ng pro g Fischgewicht, beibehalten wird.

3. Verfahren nach Anspruch 2, bei dem das Trägermaterial so ausgewählt und die Anfangskonzentration des Somatotropins so eingestellt wird, daß die gesamte fortwährende Freisetzung in vivo bei einer Temperatur zwischen 1 ° u nd 16° erfolgt, wobei die Kinetik der Freisetzung so beschaffen ist, daß die pST-Menge in dem Fisch während dieses Zeitraumes größer als 5 ng pro ml Blutserum, bestimmt durch Radioimmunoassay (RIA), beschrieben von Bolton et al, Gen. Comp. Endocrinol. (1986) 62, 230-238, ist.

4. Verfahren gemäß eines jeden der Ansprüche 1 bis 3, bei dem die Zusammensetzung 50 ng bis 2 µg Somatotropin pro g Fisch enthält.

5. Verfahren gemäß eines jeden der Ansprüche 1 bis 4, bei dem das Somatotropin in diskreten, nicht-abbaubaren und abbaubaren, physiologisch verträglichen polymeren Systemen, Liposomen oder osmotischen Pumpen eingeschlossen ist.

6. Verfahren nach Anspruch 5, bei dem das Trägermaterial in Form von Teilchen mit einem mittleren Teilchendurchmesser im Bereich von 100 nm bis 500 µm, vorzugsweise von 500 nm bis 1 µm, vorliegt.

7. Verfahren nach Anspruch 6 oder 7, bei dem der teilchenförmige Träger von einem Poly(d,l-Lactid-co-glycolid) gebildet ist.

8. Verfahren gemäß eines jeden der Ansprüche 5 bis 7, bei dem die Teilchen 0,01 bis 10 % (w/w) Somatotropin enthalten.

9. Verfahren gemäß eines jeden der Ansprüche 1 bis 8, bei dem das Somatotropin ein rekombinantes Somatotropin mit im wesentlichen der gleichen Aminosäuresequenz und der gleichen Aktivität wie das natürliche Somatotropin ist, insbesondere das Salmonidensomatotropin, das der salmoniden Art, an die die Zusammensetzung verabreicht wird, entspricht.

10. Verfahren nach Anspruch 9, bei dem die Menge des Somatotropins auf eine Menge von 50 ng bis 2 µg pro g der Fische, an die die Somatotropinzusammensetzung verabreicht wird, eingestellt wird.

11. Verfahren gemäß eines jeden der Ansprüche 1 bis 9, bei dem die Zusammensetzung in flüssiger Form oder in ausreichend flüssigem Zustand oder in einer dispergierten Form vorliegt, damit diese in die Fische injiziert werden kann, und bei dem die Verabreichung durch Injektion erfolgt.

12. Verfahren gemäß eines jeden der Ansprüche 1 bis 9, bei dem die Zusammensetzung auf oralem Wege verabreicht wird.

13. Verfahren nach Anspruch 12, bei dem die Zusammensetzung den Salmoniden im Gemisch mit Futter zugeführt wird.

14. Verfahren nach Anspruch 13 in Kombination mit demjenigen des Anspruchs 9, bei dem die Zusammensetzung in Form eines nichtpathogenen Mikroorganismus als Wirtszelle, in dem das genannte Somatotropin gebildet worden ist, vorliegt und wobei das rekombinante Somatotropin vorzugsweise 0,1 % (w/w) des Gesamtproteins beträgt.

## Revendications

1. Procédé pour contrôler l'adaptation à l'eau de mer des salmonidés d'élevage, lorsque ceux-ci sont transférés de l'eau douce à l'eau de mer et doivent subir la smoltification, qui comprend l'administration à ces salmonidés d'une composition de somatotropine, de préférence une somatotropine de salmonidés, en association avec un matériau de support acceptable du point de vue physiologique choisi de façon à réaliser une encapsulation, une adsorption ou un piègeage réversible de cette somatotropine et à permettre une libération continue et retardée in vivo de la somatotropine, dans lequel les quantités de somatotropine et lorsque cela convient, de support, sont ajustées de façon à permettre un taux de survie d'au moins 50% des salmonidés maintenus initialement dans l'eau douce, un mois après la fin de l'administration de la composition ou de la smoltification, quelle que soit la dernière.

2. Procédé suivant la revendication 1, dans lequel la quantité de somatotropine est ajustée à la fois en fonction de la taille des salmonidés et du volume de la composition qui leur est administrée par une voie quelconque, de façon à fournir une libération continue, pendant une période d'environ sept jours, et à provoquer le maintien de la concentration de la somatotropine dans le sérum de ces salmonidés à un niveau pratiquement permanent, par exemple supérieur à 3 ng, tel que par exemple 5 ng/g de poisson pendant cette période.

3. Procédé suivant la revendication 2, dans lequel le matériau de support est choisi et dans lequel la concentration initiale de somatotropine est ajustée de façon à fournir cette libération continue ci- dessus in vivo à une température comprise entre 1° et 16°, les cinétiques de la libération étant telles que la quantité de pST dans le poisson pendant cette période dépasse 5 ng/ml de sérum sanguin, telle qu'elle est mesurée par le radioim- munoessai (RIA) décrit par Bolton et al., Gen. Comp. Endocrinol. (1986), 62, 230-238.

4. Procédé suivant l'une quelconque des revendications 1 à 3, dans lequel cette composition contient de 50 ng à 2 µg de somatotropine par gramme de poisson.

5. Procédé suivant l'une quelconque des revendications 1 à 4, dans lequel cette somatotropine est piégée dans des systèmes polymères acceptables du point de vue physiologique, particulaires, non dégradables et dégradables, des liposomes ou des pompes osmotiques.

6. Procédé suivant la revendication 5, dans lequel ce matériau de support est sous la forme de particules ayant en particulier un diamètre moyen compris entre 100 nm et 500 µm, de préférence entre 500 nm et 1 µm.

7. Procédé suivant les revendications 6 ou 7, dans lequel le matériau de support est formé d'un poly(d,l-lactide-co-glycol ide).

8. Procédé suivant l'une quelconque des revendications 5 à 7, dans lequel les particules contiennent de 0,01 à 10% (p/p) de somatotropine.

9. Procédé suivant l'une quelconque des revendications 1 à 8, dans lequel cette somatotropine est une somatotropine recombinante ayant pratiquement la même séquence d'acides aminés et la même activité que la somatotropine naturelle, en particulier la somatotropine de salmonidé correspondant à l'espèce de salmonidé à laquelle est administrée cette composition.

10. Procédé suivant la revendication 9, dans lequel la quantité de somatotropine est ajustée à une quantité comprise entre 50 ng et 2 µg par gramme de poisson auquel est administrée la composition de somatotropine.

11. Procédé suivant l'une quelconque des revendications 1 à 9, dans lequel cette composition est sous une forme liquide ou une forme suffisamment fluide ou dispersée pour permettre son injection dans le poisson et dans lequel cette administration est faite par injection.

12. Procédé suivant l'une quelconque des revendications 1 à 9, dans lequel cette composition est administrée par voie orale.

13. Procédé suivant la revendication 12, dans lequel cette composition est fournie à ces salmonidés en mélange avec de la nourriture.

14. Procédé suivant la revendication 13 en association avec la revendication 9, dans lequel cette composition est sous la forme du microorganis- me non pathogène servant de cellule hôte dans lequel cette somatotropine recombinante a été formée et dans lequel cette somatotropine recombinante représente de préférence 0,1% (p/p) de la protéine totale.
